# FASCICULE DE BREVET EUROPEEN

(11) **EP 3 013 924 B1**
(45) Date de publication et mention de la délivrance du brevet: **11.12.2019**
(21) Numéro de dépôt: 14733166.4
(22) Date de dépôt: 25.06.2014
(51) Int. Cl.: C10G 70/04, F25J 3/06, C07C 11/04, B01D 53/00, B01D 53/047, B01D 53/14, C07C 7/00, C07C 7/04, F25J 3/02

(54) **PROCÉDÉ DE RÉCUPÉRATION D'UN COURANT D'ÉTHYLÈNE À PARTIR D'UN COURANT DE CHARGE RICHE EN MONOXYDE DE CARBONE**
VERFAHREN ZUR RÜCKGEWINNUNG EINES ETHYLENSTROMS AUS EINEM KOHLENMONOXIDREICHEN ZUFUHRSTROM
METHOD FOR RECOVERING AN ETHYLENE STREAM FROM A CARBON MONOXIDE RICH FEED STREAM

(30) Priorité: 25.06.2013 FR 1356061
(43) Date de publication de la demande: 04.05.2016
(73) Titulaire: Technip France, 92400 Courbevoie (FR)
(72) Inventeur: DESTOUR, Bruno, 92500 Rueil Malmaison (FR); SIMON, Yvon, 78570 Andresy (FR); DADOU, Aurélia, 92800 Puteaux (FR)
(74) Mandataire: Lavoix
(86) Numéro de dépôt international: PCT/EP2014/063424
(87) Numéro de publication internationale: WO 2014/207053

(56) Documents cités:
- FR-A1- 2 817 767
- US-A1- 2002 007 101

## Description

La présente invention concerne un procédé de récupération d'un courant d'éthylène à partir d'un courant de charge, selon le préambule de la revendication 1.

Un tel procédé est destiné notamment à traiter un courant de charge destiné à la production d'éthylène, ce courant de charge provenant de sources non conventionnelles d'éthylène. Le courant de charge comprend une quantité importante de monoxyde de carbone.

La production d'oléfines légères, telles que l'éthylène, présente une importance économique considérable. En effet, ces produits sont des précurseurs de base de l'industrie des polymères et sont utilisés pour former des matières plastiques utilisées de manière large dans l'industrie pour fabriquer notamment des biens de consommation, des pièces pour des véhicules de transport, ou encore des pièces pour bâtiments.

À l'heure actuelle, la majeure partie des oléfines légères produites industriellement proviennent de la pyrolyse d'hydrocarbures à haute température, notamment en présence de vapeur.

Le courant de charge alimentant la pyrolyse comprend essentiellement de l'éthane, du propane, du butane, du naphta et/ou du gazole, seul ou en mélange.

En sortie de la pyrolyse, le gaz craqué comprend un mélange d'eau, d'éthylène, d'éthane, d'hydrogène, de méthane et d'autres composés hydrocarbonés en proportions variables. Dans ce type de production, il est usuel d'obtenir un taux de récupération et une pureté pour les oléfines produites qui sont très élevés. Typiquement, pour l'éthylène, le taux de récupération est supérieur à 99 %, pour une pureté supérieure à 99.5%.

Cependant, d'autres sources d'éthylène sont actuellement en cours de développement. Ces sources produisent un courant de charge de composition très différente de celui qui est obtenu usuellement à la sortie d'un vapocraqueur.

Le courant de charge peut comprendre ainsi une forte teneur molaire en monoxyde de carbone, par exemple supérieure à 10 %, voire supérieure à 20 %.

Dans ce cas, les procédés usuels de récupération et de traitement du courant de charge pour obtenir l'éthylène ne peuvent pas être utilisés, ou donnent des rendements et des taux de récupération faibles.

US 6,303,841 décrit un procédé de récupération et de concentration d'éthylène à partir d'une charge résultant d'un procédé de conversion de composés oxygénés, tels que des alcools.

Un tel procédé est efficace pour récupérer l'éthylène, mais nécessitent des équipements compliqués, et notamment une pluralité de colonnes de distillation.

FR2817767 décrit un procédé de récupération d'éthylène produit par pyrolyse d'hydrocarbures.

Un but de l'invention est d'obtenir un procédé de récupération d'éthylène, à partir d'un courant de charge provenant d'une source non conventionnelle, le procédé étant simple à mettre en oeuvre, tout en garantissant un taux de récupération et une pureté en éthylène très élevés.

À cet effet, l'invention a pour objet un procédé selon la revendication 1.

Le procédé selon l'invention peut comprendre l'une ou plusieurs des caractéristiques des revendications 2 à 11 ou la caractéristique suivante, prise(s) isolément ou suivant toute combinaison techniquement possible :
- la température de la ou de chaque fraction d'alimentation de colonne est supérieure à -40°C.

L'invention sera mieux comprise à la lecture de la description qui va suivre, donnée uniquement à titre d'exemple, et faite en se référant aux dessins annexés, sur lesquelles :
- la figure 1 est un schéma synoptique fonctionnel d'une première installation destinée à la mise en oeuvre d'un premier procédé de récupération d'éthylène selon l'invention ;
- la figure 2 est une vue d'un détail d'une première variante de la première installation ;
- la figure 3 est une vue d'un détail d'une deuxième variante de la première installation ;
- la figure 4 est une vue d'un détail d'une troisième variante de la première installation ;
- la figure 5 est un schéma synoptique fonctionnel d'une deuxième installation destinée à la mise en oeuvre d'un deuxième procédé de récupération d'éthylène selon l'invention ;
- la figure 6 est une vue d'un détail d'une première variante de la deuxième installation.

Dans tout ce qui suit, une même référence désigne un courant circulant dans une conduite et la conduite qui transporte ce courant. Par ailleurs, sauf indication contraire, les pourcentages sont des pourcentages molaires, les températures et les pressions s'entendent respectivement en degrés Celsius et en kilogramme-force par centimètre carré (kgf/cm²) relatifs.

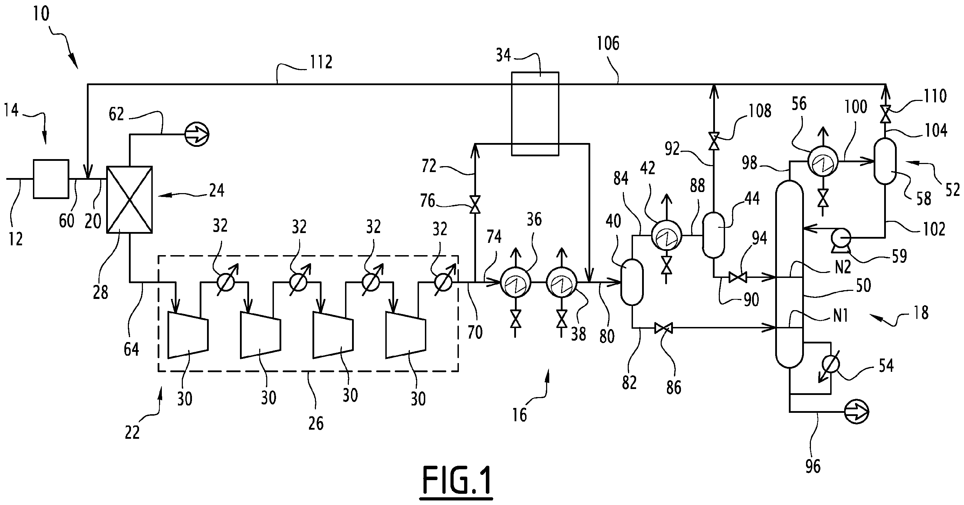

Une première installation 10 de récupération d'éthylène à partir d'un courant de charge gazeux 12 est illustrée par la figure 1.

Le courant de charge 12 est obtenu à partir de sources non conventionnelles d'éthylène, et non à partir d'un procédé de craquage d'hydrocarbures à haute température en présence de vapeur.

Le courant de charge 12 comporte une teneur molaire en éthylène supérieure à 20 % et comprise entre 20 % et 80 %, avantageusement entre 40 % et 60 %. Il comporte une teneur molaire en monoxyde de carbone supérieure à 20 %, et comprise entre 20 % et 80 %, typiquement entre 40 % et 60 %.

La teneur molaire en méthane dans le courant de charge 12 est inférieure à 20 %. La teneur molaire en hydrogène dans le courant de charge 12 est typiquement inférieure à 10 %.

Le courant de charge 12 comporte des impuretés, tels que des gaz acides, notamment du dioxyde de carbone (CO₂) et potentiellement du sulfure d'hydrogène (H₂S) ou d'autres impuretés, telles que des composés oxygénés et de l'eau.

L'installation 10 comporte un ensemble 14 de traitement du gaz de charge 12 destiné à former un courant de charge purifié, un ensemble d'élimination 22 destiné à préfractionner le gaz purifié, un ensemble 16 de refroidissement et de condensation au moins partielle d'un gaz traité obtenu à partir du courant de charge, et un ensemble 18 de distillation.

L'ensemble de traitement 14 est propre à éliminer les impuretés présentes dans le courant de charge 12 pour former au moins en partie un courant intermédiaire 20 riche en monoxyde de carbone.

À cet effet, l'ensemble 14 de traitement est propre à engendrer un courant de charge 60 purifié ne comprenant plus d'impuretés, tels que des gaz acides, notamment du dioxyde de carbone (CO₂) et potentiellement du sulfure d'hydrogène (H₂S) ou d'autres impuretés, telles que des composés oxygénés autre que le monoxyde de carbone (CO) et de l'eau.

L'ensemble de traitement 14 comporte par exemple une tour de lavage à la soude caustique propre à éliminer les gaz acides, ou encore une tour de lavage à l'amine.

L'ensemble de traitement 14 comporte également par exemple des tamis moléculaires de séchage propre à éliminer l'eau. L'ensemble de traitement 14 peut également contenir des lits de catalyseurs ou de pièges pour enlever les impuretés présentes dans le gaz, notamment des métaux lourds.

Comme on le verra plus bas, le courant intermédiaire 20 riche en monoxyde de carbone est formé dans l'ensemble de traitement 14 à partir du courant de charge purifié 60.

L'ensemble 22 d'élimination est destiné notamment à éliminer le monoxyde de carbone contenu dans le courant intermédiaire 20.

Dans cet exemple, l'ensemble d'élimination 22 est interposé entre l'ensemble de traitement 14 et l'ensemble de condensation 16.

Dans l'exemple représenté sur la figure 1, l'ensemble d'élimination 22 comporte un dispositif 24 d'élimination à basse pression du monoxyde de carbone contenu dans le courant intermédiaire 20, et un dispositif 26 de compression destiné à former le courant de gaz traité.

De préférence, dans cette variante, le dispositif d'élimination 24 comporte un appareil 28 d'adsorption du monoxyde de carbone par modulation de pression, désigné par le terme anglais « pressure swing adsorber » ou « PSA ».

Un tel appareil 28 opère à une pression de purge basse, par exemple inférieure à 10 kgf/cm² et notamment comprise entre 0,1 kgf/cm² et 5 kgf/cm². Il est propre à produire, à partir du courant intermédiaire 20, un courant 62 pauvre en éthylène, comportant moins de 1 % en moles de l'éthylène contenu dans le courant intermédiaire 20, et plus de 60 % en moles du monoxyde de carbone contenu dans le courant intermédiaire 20, avantageusement plus de 80%.

L'appareil 28 d'adsorption par modulation de pression comporte au moins deux enceintes opérant tour à tour dans des conditions d'adsorption de l'éthylène sur un support, à relativement haute pression, et de désorption de l'éthylène, à relativement basse pression, pour relâcher l'éthylène adsorbé sur le support.

Le support d'adsorption comporte par exemple un ou plusieurs lits de tamis moléculaires, notamment des lits de zéolites, et/ou d'aluminosilicates et/ou de charbon microporeux.

Le dispositif de compression 26 comporte un compresseur lui-même constitué d'une pluralité d'étages de compression 30 montés en série, et une pluralité de réfrigérants 32, montés à la sortie de chaque étage de compression 30 pour refroidir le gaz comprimé issu du compresseur 30.

L'ensemble de refroidissement et de condensation 16 comporte au moins un échangeur thermique amont 34, 36, 38, pour former un courant de gaz amont partiellement condensé et un séparateur amont 40, pour séparer le courant de gaz amont.

L'ensemble de refroidissement et de condensation 16 comporte dans cet exemple au moins un échangeur thermique aval 42, pour former un courant de gaz aval partiellement condensé, et un séparateur aval 44 pour séparer le courant de gaz aval.

Dans cet exemple, l'ensemble de refroidissement et de condensation 16 comporte un premier échangeur thermique amont 34, un deuxième échangeur thermique amont 36, et un troisième échangeur thermique amont 38.

Le deuxième échangeur thermique amont 36 et le troisième échangeur thermique amont 38 sont montés en série. Ils sont raccordés à une boucle de refroidissement utilisant un réfrigérant dans un cycle de réfrigération (non représenté). Le réfrigérant est avantageusement un hydrocarbure tel que du propylène.

L'ensemble de distillation 18 comporte une colonne de distillation 50, un condenseur de tête 52, et un rebouilleur de fond 54.

Dans cet exemple, la colonne 50 est une colonne de distillation avec rebouillage (désignée par le terme anglais « reboiled stripper column »).

La colonne de distillation 50 est propre à opérer à une pression comprise entre 10 kgf/cm² et 40 kgf/cm², notamment entre 25 kgf/cm² et 40 kgf/cm², de préférence entre 30 kgf/cm² et 40 kgf/cm².

Elle comporte des plateaux ou un garnissage. Elle comporte par exemple plus de 6 plateaux théoriques, et notamment entre 10 plateaux théoriques et 20 plateaux théoriques.

Le condenseur de tête 52 comporte un échangeur thermique de tête 56, un séparateur de tête 58, et une pompe 59 de reflux.

Un premier procédé de récupération d'un courant d'éthylène à partir d'un courant de charge 12, mis en oeuvre dans la première installation 10, va maintenant être décrit.

Initialement, le courant de charge 12, maintenu à une pression comprise avantageusement entre 10 kgf/cm² et 40 kgf/cm², est introduit dans l'ensemble de traitement 14. Il présente avantageusement une température comprise entre 10°C et 50°C. Le courant de charge 12 est riche en éthylène et en monoxyde de carbone. Il présente de préférence la composition décrite plus haut.

Un courant de charge 60 purifié, dépourvu d'impuretés, est extrait de l'ensemble de traitement 14.

Le courant de charge purifié 60 présente une teneur molaire en gaz acides, tel que du dioxyde de carbone (CO₂) et du sulfure d'hydrogène (H₂S)] inférieure à 1 ppm (0,0001% molaire), et en eau inférieure à inférieure à 1 ppm (0,0001% molaire)
Le courant de charge purifié 60 forme au moins partiellement le courant intermédiaire 20.

La teneur molaire en éthylène dans le courant intermédiaire 20 est supérieure à 20 %, et est notamment comprise entre 20 % et 80 %, de préférence entre 40 % et 60 %. La teneur molaire en monoxyde de carbone dans le courant intermédiaire 20 est supérieure à 20 % et est notamment comprise entre 20 % et 80 %, de préférence entre 40 % et 60 %.

Le courant intermédiaire 20 est alors introduit dans le dispositif d'élimination 24. Un courant 62 riche en monoxyde de carbone est alors extrait en continu et à haute pression du dispositif d'élimination 24. Un courant 64 pauvre en monoxyde de carbone est simultanément extrait en continu et à basse pression du dispositif d'élimination 24.

À cet effet, le courant intermédiaire 20 passe successivement dans l'une des enceintes de l'appareil d'adsorption 28, pour permettre l'adsorption de l'éthylène contenu dans le courant 20 sur le support contenu dans l'enceinte et la formation du courant riche en monoxyde de carbone 62. Simultanément, l'éthylène chargé sur le support contenu dans l'autre des enceintes désorbe et forme le courant pauvre en monoxyde de carbone 64.

Le courant riche en monoxyde de carbone 62 présente une pression supérieure à 10 kgf/cm² et notamment comprise entre 20 kgf/cm² et 40 kgf/cm². Il comporte plus de 60 % molaire du monoxyde de carbone contenu dans le courant intermédiaire 20, avantageusement plus de 80% molaire.

Le courant pauvre en monoxyde de carbone 64 présente une pression inférieure à 10 kgf/cm² et notamment comprise entre 0,1 kgf/cm² et 5 kgf/cm². Il comporte plus de 99 % molaire de l'éthylène contenue dans le courant intermédiaire 20, et avantageusement moins de 20 % en moles de monoxyde de carbone.

Le courant pauvre en monoxyde de carbone 64 est ensuite introduit dans le dispositif de compression 26. Il est alors comprimé dans les étages de compression successifs 30 jusqu'à la pression d'opération de la colonne 50, en étant refroidi à chaque étage de compression dans un réfrigérant 32.

Le courant de gaz traité 70 récupéré à la sortie de l'appareil 26 présente avantageusement une pression comprise entre 10 kgf/cm² et 40 kgf/cm², notamment entre 25 kgf/cm² et 40 kgf/cm². La pression du flux 70 est contrôlée par une vanne 108 située en aval du séparateur aval 44.

La température du courant de gaz traité 70 est par exemple supérieure à 10°C, notamment comprise entre 20°C et 50°C.

Le courant de gaz traité 70 est ensuite introduit dans l'ensemble de condensation 16 pour y être au moins partiellement condensé dans les échangeurs 36, 38, 34.

Dans cet exemple, le courant de gaz traité 70 est séparé en une première fraction 72 et en une deuxième fraction 74.

La première fraction 72 est introduite dans le premier échangeur thermique 34 pour y être refroidie jusqu'à une température inférieure à -10° C, et notamment comprise entre -15° C et -25° C.

La deuxième fraction 74 est introduite successivement dans le deuxième échangeur thermique 36 et dans le troisième échangeur thermique 38 le pour y être refroidie par échange thermique avec le réfrigérant circulant dans le cycle de réfrigération externe, par exemple avec du propylène, par vaporisation du réfrigérant.

La deuxième fraction 74 est refroidie jusqu'à une température inférieure à -10°C et notamment comprise entre -15 °C et -25 °C.

Le rapport du débit molaire de la première fraction 72 à la deuxième fraction 74 est piloté par une vanne 76, en fonction des frigories disponibles dans le premier échangeur thermique 34.

La première fraction 72 refroidie et la deuxième fraction 74 refroidie sont ensuite regroupées pour former un courant amont 80 au moins partiellement condensé.

La teneur molaire en liquide dans le courant amont 80 est par exemple supérieure à 40%, et est notamment comprise entre 50% et 70%. Le courant amont 80 est ensuite introduit dans le séparateur amont 40 pour y être séparé en une fraction liquide amont 82 et en une fraction gazeuse amont 84.

La fraction liquide amont 82 forme une première fraction d'alimentation de la colonne 50 qui est introduite dans la colonne 50 à un premier niveau N1 à travers une vanne de commande de débit 86.

La fraction gazeuse amont 84 est ensuite introduite dans le premier échangeur thermique aval 42 pour y être refroidie et partiellement condensée, par échange thermique avec un réfrigérant circulant dans un cycle de réfrigération externe, et former un courant aval 88.

La température du courant aval 88 est inférieure à -20 °C et est notamment comprise entre -25 °C et -40 °C.

La teneur molaire en liquide dans le courant aval 88 est par exemple supérieure à 30%, et est notamment comprise entre 40% et 60%.

Le courant aval 88 est ensuite introduit dans le séparateur aval 44 pour y être séparé en une fraction liquide aval 90 et en une fraction gazeuse aval 92.

La fraction liquide aval 90 forme une deuxième fraction d'alimentation de la colonne 50 qui est introduite dans la colonne 50 à un deuxième niveau N2 situé au-dessus du premier niveau N1, à travers une vanne de commande de débit 94.

La pression de la colonne 50 est inférieure à 40 kgf/cm², et est notamment comprise entre 25 kgf/cm² et 40 kgf/cm². La pression de la colonne 50 est contrôlée par une vanne 110 située en aval du séparateur de tête 58.

La colonne 50 est chauffée à l'aide d'un rebouilleur de fond 54 dans lequel circule un courant qui peut être typiquement un réfrigérant à condenser circulant dans un cycle de réfrigération externe destinée à alimenter l'un des échangeurs 36, 38, 42.

Un courant de pied 96 riche en éthylène est récupéré au pied de la colonne 50. Le courant de pied 96 présente une teneur molaire en éthylène supérieure à 99,5% et une teneur molaire en monoxyde de carbone inférieure à 0,0001 % molaire (1 ppm molaire). Le courant de pied 96 contient plus de 99 % molaire de l'éthylène contenu dans le courant de charge 12.

Ainsi, le courant de pied 96 peut être utilisé directement dans une unité de production de polymère, sans avoir à être distillé à nouveau.

Un courant de tête 98 pauvre en éthylène est extrait en tête de la colonne 50.

Le courant de tête 98 et partiellement condensé dans l'échangeur de tête 56 par échange thermique avec un réfrigérant (typiquement du propylène se vaporisant) circulant dans une boucle de réfrigération traditionnelle d'un cycle de réfrigération fermé.

Le courant de tête 100 partiellement condensé est ensuite introduit dans le séparateur de tête 58 pour y être séparé en une fraction liquide de tête 102 et en une fraction gazeuse de tête 104.

La fraction liquide de tête 102 est pompée à l'aide de la pompe 59 en reflux dans la colonne 50.

La fraction gazeuse de tête 104 est ensuite mélangée à la fraction gazeuse aval 92 issue du séparateur aval 44 pour former un flux aval 106 de tête, issu du courant de tête 98.

La température du flux aval 106 en amont de l'échangeur aval 34 est par exemple comprise entre -25°C et -40°C. La pression du flux aval 106 est égale à la pression du flux 60 plus la perte de charge générée dans l'échangeur thermique 34. Cette pression est par exemple comprise entre 20 kgf/cm² et 40 kgf/cm².

Le flux aval 106 est ensuite introduit dans le premier échangeur thermique 34 pour être réchauffé par échange thermique avec la première fraction 72 du courant de gaz traité 70.

Le flux aval réchauffé 112 issu de l'échangeur 34 présente une température supérieure à 0°C. Il est ensuite réintroduit au moins partiellement dans le courant de charge traité 60 pour former le courant intermédiaire 20.

Le procédé selon l'invention permet donc, d'une manière simple et particulièrement économique, de traiter un courant de charge 12 provenant d'une source non conventionnelle d'éthylène, pour en extraire la totalité de l'éthylène et produire un courant 96 répondant aux spécifications pour la production de polymère.

Ce résultat est obtenu à l'aide d'une seule étape de distillation réalisée dans une seule colonne 50, à l'aide d'un ensemble d'élimination 22 du monoxyde de carbone simple à utiliser.

La récupération de l'éthylène dans le courant de charge 12 est quasi totale, par exemple supérieure à 99 %, et l'éthylène obtenu présente une pureté notamment supérieure à 99,5 %.

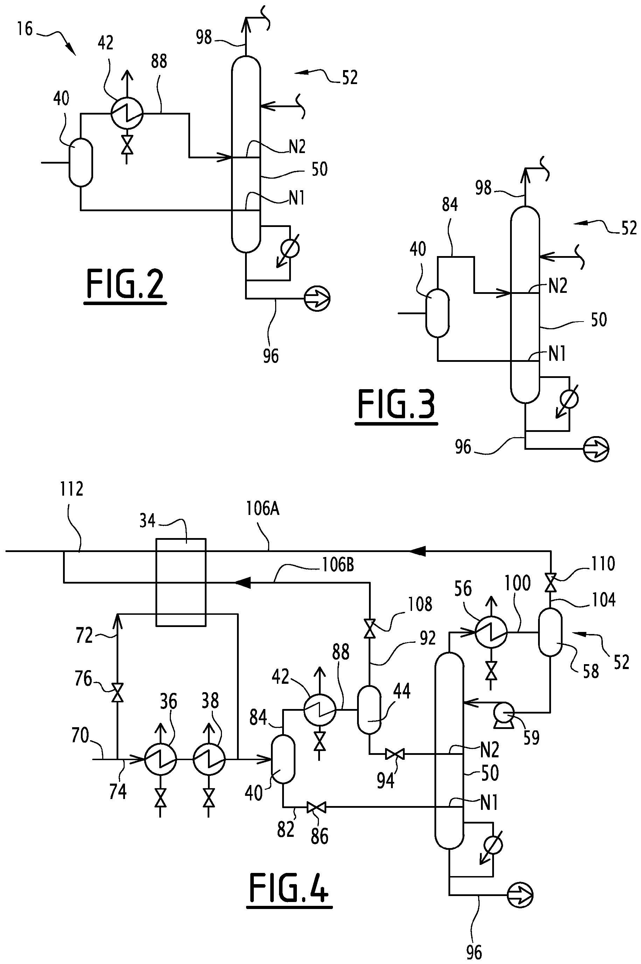

Dans une première variante de la première installation 10, représentée partiellement sur la figure 2, l'ensemble de condensation 16 est dépourvu de séparateur aval 44.

Le courant aval 88 refroidi et au moins partiellement condensé dans l'échangeur aval 42 est introduit directement dans la colonne 50 un niveau N2, situé au-dessus du niveau N1 d'introduction de la première fraction d'alimentation de colonne.

Le procédé de récupération mis en oeuvre dans cette variante d'installation 10 est par ailleurs analogue à celui mis en oeuvre dans la première installation 10.

Dans une deuxième variante de la première installation 10, représentée partiellement sur la figure 3, l'ensemble de condensation 16 et est également dépourvu de séparateur aval 44 et est dépourvu d'échangeur thermique aval 42.

La fraction gazeuse amont 84 issue du séparateur amont 40 est alors introduite directement dans la colonne 50 pour former la deuxième fraction d'alimentation de colonne.

Le procédé de récupération mis en oeuvre dans cette variante d'installation 10 est par ailleurs analogue à celui mis en œuvre dans la première installation 10.

Dans une troisième variante de la première installation 10, représentée partiellement sur la figure 4, la fraction gazeuse de tête 104 issue de la vanne 110, et la fraction gazeuse aval 92 issue de la vanne 108 forment respectivement un premier flux aval 106A et un deuxième flux aval 106B qui sont introduits séparément dans le premier échangeur thermique 34, pour y être réchauffés, avant d'être mélangés l'un à l'autre en aval du premier échangeur thermique 34.

Le procédé de récupération mis en oeuvre dans cette variante d'installation 10 est par ailleurs analogue à celui mis en œuvre dans la première installation 10.

Dans une variante (non représentée), l'échangeur thermique de tête 56 est un échangeur thermique vertical disposé dans la colonne 50.

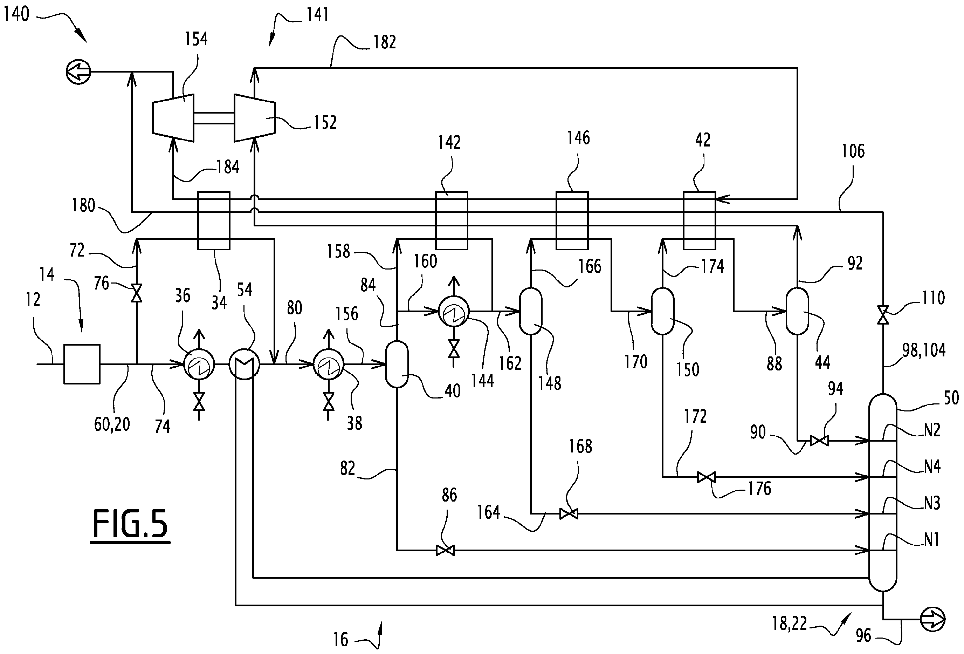

Une deuxième installation 140 est illustrée par la figure 5.

Comme la première installation 10, la deuxième installation 140 comporte un ensemble 14 de traitement du gaz de charge 12 destiné à former un gaz traité, un ensemble 16 de refroidissement de condensation au moins partielle du gaz traité, et un ensemble 18 de distillation.

L'ensemble de traitement 14 est propre à engendrer un courant intermédiaire 20 riche en monoxyde de carbone.

Toutefois, à la différence de l'installation 10, l'ensemble 22 d'élimination du monoxyde de carbone contenu dans le courant intermédiaire 20 est formé directement par l'ensemble de distillation 18.

L'installation 140 comporte en outre un ensemble 141 de refroidissement supplémentaire du courant de charge traité 20.

L'ensemble de traitement 14 est analogue à celui de la première installation 10. Il ne sera pas décrit plus en détail.

Comme pour la première installation 10, l'ensemble de refroidissement et de condensation 16 comporte un étage amont comprenant au moins un échangeur thermique amont 34, 36, 38, pour former un courant de gaz amont partiellement condensé et un séparateur amont 40, pour séparer le courant de gaz amont.

L'ensemble de condensation 16 comporte également un étage aval comprenant au moins un échangeur thermique aval 42, pour former un courant de gaz aval partiellement condensé, et un séparateur aval 44 pour séparer le courant de gaz aval.

À la différence de la première installation 10, l'ensemble de condensation 16 de la deuxième installation comporte en outre un étage intermédiaire comprenant des échangeurs thermiques intermédiaires 142, 144, 146, et des séparateurs intermédiaires 148, 150 disposés respectivement en aval des échangeurs thermiques intermédiaires 142, 144 et de l'échangeur thermique intermédiaire 146.

Dans cet exemple, le premier échangeur thermique intermédiaire 142 est monté en parallèle du deuxième échangeur thermique intermédiaire 144.

Le premier échangeur thermique intermédiaire 142 est propre à être refroidi par chauffage d'un flux aval 106 obtenu à partir d'un courant de tête 98 formé dans l'ensemble de distillation 18.

Le premier séparateur intermédiaire 148 est interposé entre les échangeurs 142, 144 et l'échangeur 146.

Le deuxième échangeur thermique intermédiaire 144 est propre à être refroidi par l'intermédiaire de la vaporisation d'un réfrigérant circulant dans un cycle fermé de réfrigération (non représenté). Le réfrigérant est par exemple de l'éthylène.

Le troisième échangeur thermique intermédiaire 146 est propre à être refroidi par chauffage d'un flux aval 106 obtenu à partir d'un courant de tête 98 formé dans l'ensemble de distillation 18.

Dans l'exemple représenté sur la figure 5, l'ensemble de distillation 18 est dépourvu de condenseur de tête 52. La colonne de distillation 50 est une colonne d'absorption avec rebouillage.

Le rebouilleur 54 est placé en aval du premier échangeur thermique amont 36, et en amont du deuxième échangeur thermique amont 38, pour être mis en relation d'échange thermique avec la deuxième fraction 74.

L'ensemble de refroidissement supplémentaire 141 comporte une turbine de détente dynamique 152 couplée à un compresseur 154. La turbine de détente dynamique 152 est propre à recevoir au moins une partie du flux gazeux aval pour le détendre et le faire circuler à travers les échangeurs thermiques 34, 142, 146, 42 et fournir des frigories pour le refroidissement du courant de gaz traité 20.

Un deuxième procédé selon l'invention, mise en oeuvre dans la deuxième installation 140 va maintenant être décrit.

Initialement, le courant de charge 12, maintenu à une pression comprise avantageusement entre 10 kgf/cm² et 40 kgf/cm², notamment entre 20 kgf/cm² et 30 kgf/cm², est introduit dans l'ensemble de traitement 14. Il présente avantageusement une température comprise entre 10°C et 50 °C

Le courant de charge 12 est riche en éthylène et en monoxyde de carbone. Il présente de préférence la composition décrite plus haut.

Un courant de charge 60 traité, dépourvu d'impuretés, est extrait de l'ensemble de traitement 14. Le courant de charge 60 présente une teneur molaire en gaz acides, tel que du dioxyde de carbone (CO₂) et du sulfure d'hydrogène (H₂S)] inférieure à 1 ppm molaire (0,0001% molaire), et une teneur molaire en eau inférieure à inférieure à 1 ppm molaire (0,0001% molaire)

Dans cet exemple, le courant de charge traité 60 constitue le courant intermédiaire 20 riche en monoxyde de carbone.

La teneur molaire en éthylène dans le courant intermédiaire 20 est supérieure à 20 %, et est notamment comprise entre 20 % et 80 %, de préférence entre 40 % et 60 %. La teneur molaire en monoxyde de carbone dans le courant intermédiaire 20 est supérieure à 20 % et est notamment comprise entre 20 % et 80 %, de préférence entre 40 % et 60%.

Le courant intermédiaire 20 présente avantageusement une pression comprise entre 10 kgf/cm² et 40 kgf/cm², notamment entre 20 kgf/cm² et 35 kgf/cm², en particulier inférieure à 30 kgf/cm².

La température du courant intermédiaire 20 est par exemple supérieure à 10°C, notamment comprise entre 20°C et 50°C.

Le courant intermédiaire 20 est ensuite introduit dans l'ensemble de condensation 16 pour y être au moins partiellement condensé dans les échangeurs 36, 38, 34, 54.

Dans cet exemple, le courant intermédiaire 20 est séparé en une première fraction 72 et en une deuxième fraction 74.

La première fraction 72 est introduite dans le premier échangeur thermique 34 pour y être refroidie jusqu'à une température inférieure à -30° C, et notamment comprise entre -40° C et -70° C.

La deuxième fraction 74 est introduite successivement dans le deuxième échangeur thermique 36 et dans le rebouilleur 54 pour y être refroidie respectivement par échange thermique avec le réfrigérant circulant dans le cycle de réfrigération externe, par exemple avec du propylène, et par un courant de rebouillage prélevé dans le fond de la colonne 50.

La deuxième fraction 74 est refroidie jusqu'à une température inférieure à -30°C et notamment comprise entre -40 °C et -70 °C.

Le rapport du débit molaire de la première fraction 72 à la deuxième fraction 74 est piloté par une vanne 76, en fonction des frigories disponibles dans le premier échangeur thermique 34.

La première fraction 72 refroidie et la deuxième fraction 74 refroidie sont ensuite regroupées pour former un courant amont 80 initial.

Le courant amont initial 80 et ensuite introduit dans le troisième échangeur thermique 38 pour former un courant amont refroidi 156 jusqu'à une température inférieure à -40 °C, et notamment comprise entre -50 °C et -80 °C.

La teneur molaire en liquide dans le courant amont refroidi 156 est par exemple supérieure à 20%, et est notamment comprise entre 25% et 50%.

Le courant amont refroidi 156 est ensuite introduit dans le séparateur amont 40, pour y être séparé en une fraction liquide amont 82 et en une fraction gazeuse amont 84.

La fraction liquide amont 82 forme une première fraction d'alimentation de la colonne 50, qui est introduite dans la colonne 50 à un premier niveau N1 à travers une vanne de commande de débit 86.

La fraction gazeuse amont 84 est séparée en un premier flux gazeux amont 158 introduit dans le premier échangeur thermique intermédiaire 142 et en un deuxième flux gazeux amont 160 introduit dans le deuxième échangeur thermique intermédiaire 144.

Le premier flux gazeux amont 158 et le deuxième flux gazeux amont 160 sont refroidis chacun jusqu'à une température inférieure à -70 °C, et notamment comprise entre -80 °C et -100 °C, avant d'être remélangés pour former un premier courant intermédiaire 162 partiellement condensé.

La teneur molaire en liquide dans le premier courant intermédiaire partiellement condensé 162 est par exemple supérieure à 15%, et est notamment comprise entre 20% et 35%.

Le premier courant intermédiaire partiellement condensé 162 est ensuite introduit dans le premier séparateur intermédiaire 148 pour y être séparée en une première fraction liquide intermédiaire 164 et en une première fraction gazeuse intermédiaire 166.

La première fraction liquide intermédiaire 164 forme une troisième fraction d'alimentation de la colonne 50, qui est introduite dans la colonne 50 à un troisième niveau N3 situé au-dessus du premier niveau N1, à travers une vanne de commande de débit 168.

La première fraction gazeuse intermédiaire 166 est introduite dans le deuxième échangeur thermique intermédiaire 146 pour former un deuxième courant intermédiaire 170 partiellement condensé et refroidi jusqu'à une température inférieure à -90 °C et notamment comprise entre -100 °C et -130 °C.

Le deuxième courant intermédiaire 170 est ensuite introduit dans le deuxième séparateur intermédiaire 150 pour y être séparé en une deuxième fraction liquide intermédiaire 172 et en une deuxième fraction gazeuse intermédiaire 174.

La deuxième fraction liquide intermédiaire 172 forme une quatrième fraction d'alimentation de la colonne 50 qui est introduite dans la colonne 50 à un quatrième niveau N4 situé au-dessus du troisième niveau N3, à travers une vanne de commande de débit 176.

La deuxième fraction gazeuse intermédiaire 174 est introduite dans le premier échangeur thermique aval 42 pour y être refroidie jusqu'à une température inférieure à - 120 °C, et notamment comprise entre -125 °C et -150 °C et former un courant aval 88 refroidi et partiellement condensé.

La teneur molaire en liquide dans le courant aval 88 est par exemple supérieure à 1%, et est notamment comprise entre 1% et 20%. Le courant aval 88 est ensuite introduit dans le séparateur aval 44 pour y être séparé en une fraction liquide aval 90 et en une fraction gazeuse aval 92.

La fraction liquide aval 90 forme une deuxième fraction d'alimentation de la colonne 50 qui est introduite dans la colonne 50 à un deuxième niveau N2 situé au-dessus du quatrième niveau N4 à travers une vanne de commande de débit 94.

La pression de la colonne 50 est inférieure à 40 kgf/cm², et est notamment comprise entre 5 kgf/cm² et 30 kgf/cm², par exemple inférieure à 15 kgf/cm².

Un courant de pied 96 riche en éthylène est récupéré au pied de la colonne 50. Le courant de pied 96 présente une teneur molaire en éthylène supérieure à 99.5% et une teneur molaire en monoxyde de carbone inférieure à 0.0001 % molaire (1 ppm molaire).. Le courant de pied 96 contient plus de 99 % molaire de l'éthylène contenu dans le courant de charge 12.

Ainsi, le courant de pied 96 peut être utilisé directement dans une unité de production de polymère, sans avoir à être distillé à nouveau.

Un courant de tête 98 pauvre en éthylène est extrait en tête de la colonne 50.

La teneur molaire en monoxyde de carbone dans le courant de tête 98 est supérieure à 70%. Le courant de tête 98 contient plus de 10% en moles du monoxyde de carbone présent dans le courant de charge 12.

Le courant de tête 98 passe à travers une vanne de contrôle de débit 110 pour former le flux aval 106, riche en monoxyde de carbone.

Le flux aval 106 est ensuite réchauffé successivement dans l'échangeur thermique aval 42, dans chaque échangeur thermique intermédiaire 146, 142, puis dans l'échangeur thermique amont 34, par échange thermique respectif avec la deuxième fraction gazeuse intermédiaire 174, la première fraction gazeuse intermédiaire 166, la première fraction gazeuse amont 158, et la première fraction 72 du courant intermédiaire 20.

Le flux aval réchauffé 180 présente ainsi une température supérieure à 10 °C à la sortie du premier échangeur thermique amont 34.

Simultanément, la fraction gazeuse aval 92 passe successivement dans l'échangeur thermique aval 42, dans chaque échangeur thermique intermédiaire 146, 142, avant d'être introduite dans la turbine de détente dynamique 152 pour y être détendue jusqu'à une pression inférieure à 10 kgf/cm².

La fraction gazeuse aval détendue 182 formée à la sortie de la turbine 152 présente une température inférieure à -120 °C et notamment comprise entre -130°C et - 150°C.

La fraction gazeuse aval détendue 182 est ensuite passée successivement dans l'échangeur thermique aval 42, dans chaque échangeur thermique intermédiaire 146, 142, puis dans l'échangeur thermique amont 34, pour y être réchauffée par échange thermique respectif avec la deuxième fraction gazeuse intermédiaire 174, la première fraction gazeuse intermédiaire 166, la première fraction gazeuse amont 158, et la première fraction 72 du courant intermédiaire 20.

La fraction gazeuse aval réchauffée 184, issue du premier échangeur thermique amont 34 est alors introduite dans le premier compresseur 154 pour y être comprimée jusqu'à une pression supérieure à 3 kgf/cm², avant d'être éventuellement mélangée avec le flux aval réchauffé 180.

Comme pour la première installation 10, la deuxième installation 140 permet d'obtenir d'une manière simple et particulièrement économique, un courant 96 d'éthylène répondant aux spécifications pour la production de polymère, à partir d'un courant de charge 12 provenant d'une source non conventionnelle et présentant une teneur élevée en monoxyde de carbone.

Ce résultat est obtenu à l'aide d'une seule étape de distillation réalisée dans une seule colonne 50, dans un ensemble de distillation 18 qui forme également un ensemble d'élimination 22 du monoxyde de carbone.

La récupération de l'éthylène dans le courant de charge 12 est quasi totale, supérieure à 99 %, et l'éthylène obtenu présent avantageusement une pureté supérieure à 99,5 %.

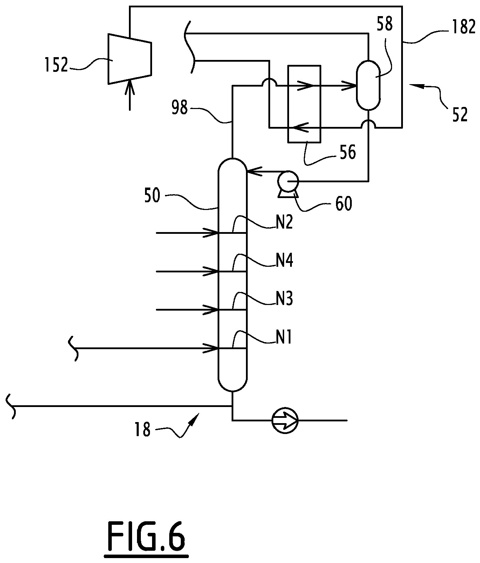

Dans une première variante de la deuxième installation, représentée schématiquement sur la figure 6, l'ensemble de distillation 18 comporte un condenseur de tête 52 analogue au condenseur de tête 52 de la première installation 10.

Le condenseur de tête 52 comporte un échangeur thermique de tête 56, un séparateur de tête 58, et une pompe 59 de reflux.

Dans l'exemple représenté sur la figure 6, l'échangeur thermique de tête 56 place en relation d'échange thermique le courant de tête 98 issu de la colonne de distillation 50 avec la fraction gazeuse aval détendue 182 issue de la turbine de détente dynamique 152, en amont du passage dans l'échangeur thermique aval 42.

Dans une autre variante, un courant de réfrigérant liquide circulant dans un cycle de réfrigération fermé ou semi-ouvert assure la production de frigories dans l'échangeur thermique de tête 56, par vaporisation du réfrigérant.

Dans une deuxième variante de la deuxième installation 140 (non représentée), au moins une fraction liquide de pied 82, 164, 172 est détendue dans une vanne de détente statique (non représentée) pour former une fraction liquide de pied détendue. La fraction liquide de pied détendue est ensuite passée successivement dans l'échangeur thermique aval 42, dans chaque échangeur thermique intermédiaire 146, 142, puis dans l'échangeur thermique amont 34, pour être réchauffée par échange thermique respectif avec la deuxième fraction gazeuse intermédiaire 174, la première fraction gazeuse intermédiaire 166, la première fraction gazeuse amont 158, et la première fraction 72 du courant intermédiaire 20.

La fraction liquide de pied réchauffée est ensuite recyclée dans le gaz de charge 12, en amont de l'ensemble de traitement 14.

Dans une autre variante (non représentée), l'énergie mécanique collectée par la turbine de détente dynamique 152 lors de la détente du flux gazeux aval 92 est dissipée à l'aide d'un frein disposé dans un bain d'huile.

Dans une autre variante (non représentée) du procédé des figures 1 à 4 le flux aval 112 est recomprimé avant d'être mélangé au courant de charge 12.

## Revendications

1. Procédé de récupération d'un courant d'éthylène (96) à partir d'un courant de charge (12), comprenant les étapes suivantes :
- traitement d'un courant de charge (12) pour obtenir un courant de gaz traité (60 ; 70) ;
- refroidissement et condensation au moins partielle du courant de gaz traité (60 ; 70) dans au moins un échangeur thermique (34, 42 ; 34, 142, 146, 42) pour former au moins une fraction d'alimentation de colonne ;
- introduction du ou de chaque fraction d'alimentation de colonne dans une colonne de distillation (50) pour récupérer, en pied de la colonne de distillation (50), un courant (96) d'éthylène et en tête, un courant de tête (98 ; 104) pauvre en éthylène ;
- réchauffage d'au moins un flux aval (106) issu du courant de tête (104) dans l'échangeur thermique (34, 42 ; 34, 142, 146, 42) ;
**caractérisé en ce que** l'étape de traitement comporte la formation d'un courant intermédiaire (20) comportant au moins 20% en moles d'éthylène et au moins 20% en moles de monoxyde de carbone, le procédé comportant une étape d'élimination du monoxyde de carbone contenu dans le courant intermédiaire (20), et **en ce que** le courant d'éthylène (96) comporte plus de 99 % en masse de l'éthylène présent dans le courant de charge (12), le courant d'éthylène (96) étant obtenu au pied de la colonne de distillation (50), sans passage par une autre colonne de distillation.

2. Procédé selon la revendication 1, **caractérisé en ce que** la pression du courant de gaz traité circulant dans le ou dans chaque échangeur thermique (34, 42 ; 34, 142, 146, 42) est inférieure à 40 kgf/cm², et est notamment comprise entre 20 kgf/cm² et 40 kgf/cm²,
la pression du courant de gaz de traité circulant dans le ou chaque échangeur thermique (34, 42 ; 34, 142, 146, 42) étant avantageusement strictement inférieure à 30 kgf/cm².

3. Procédé selon l'une quelconque des revendications précédentes, **caractérisé en ce que** le courant d'éthylène (96) comporte une teneur massique en éthylène supérieure à 99,5 %, le courant d'éthylène (96) étant obtenu au pied de la colonne de distillation (50), sans passage par une autre colonne de distillation.

4. Procédé selon l'une quelconque des revendications précédentes, **caractérisé en ce que** lors de l'étape de traitement, le courant intermédiaire (20) est introduit dans un dispositif (24) d'élimination du monoxyde de carbone sans distillation pour former un courant (62) riche en monoxyde de carbone et un courant (64) pauvre en monoxyde de carbone, la pression du courant (64) pauvre en monoxyde de carbone étant avantageusement inférieure à 5 kgf/cm², le dispositif (24) d'élimination du monoxyde de carbone comprenant avantageusement un appareil (28) d'adsorption par modulation de pression, produisant le courant (62) riche en monoxyde de carbone et le courant (64) pauvre en monoxyde de carbone.

5. Procédé selon la revendication 4, **caractérisé en ce que** le courant (64) pauvre en monoxyde de carbone est comprimé jusqu'à une pression de recompression, avantageusement inférieure à 40 kgf/cm², dans un appareil de compression (26) pour former le courant de gaz traité (70).

6. Procédé selon l'une quelconque des revendications 4 à 5, **caractérisé en ce qu'**au moins une fraction (72) du courant de gaz traité (60 ; 70) est mise en relation d'échange thermique avec le flux aval (106) dans un échangeur thermique amont (34), pour obtenir un courant amont (80) partiellement condensé,
le courant amont (80) étant introduit dans un séparateur amont (40), au moins une fraction liquide (82) issue du séparateur amont (40) étant amenée dans la colonne de distillation (50).

7. Procédé selon l'une quelconque des revendications précédentes, **caractérisé en ce que** le courant intermédiaire (20) est formé directement par le courant de gaz traité (60), puis introduit dans un ensemble (16) de condensation et de fractionnement comportant une pluralité d'échangeurs thermiques (34, 142, 146, 42) fonctionnant à des températures décroissantes pour produire une pluralité de fractions d'alimentation de la colonne de distillation (50) introduites dans la colonne de distillation (50), le courant de tête (104) issu de la colonne de distillation (50) comportant plus de 10 % en masse du monoxyde de carbone contenu dans le courant intermédiaire (20).

8. Procédé selon la revendication 7, **caractérisé en ce qu'**il comprend les étapes suivantes :
- introduction du courant de gaz traité (60) dans au moins un premier échangeur thermique amont (34) pour obtenir un courant amont (80) refroidi et partiellement condensé à une température avantageusement inférieure à -50 °C, et séparation du courant amont (80) en une fraction liquide amont (82) d'alimentation de colonne, et en une fraction gazeuse amont (84) ;
- amenée de la fraction gazeuse amont (84) dans au moins un échangeur thermique intermédiaire (142, 144, 146) pour obtenir au moins un courant intermédiaire (162, 170) refroidi et partiellement condensé à une température avantageusement inférieure à - 90 °C, et séparation du ou de chaque courant intermédiaire refroidi (162, 170) en une fraction liquide intermédiaire d'alimentation de colonne, et en une fraction gazeuse intermédiaire (166, 174) ;
- introduction d'au moins une fraction gazeuse intermédiaire (174) dans un échangeur thermique aval (42) pour obtenir un courant aval (88) refroidi et partiellement condensé à une température avantageusement inférieure à - 110 °C, et séparation du courant aval (88) en une fraction liquide aval (90) d'alimentation de colonne, et en une fraction gazeuse aval (92), les fractions liquides amont, aval et intermédiaire étant amenées dans la colonne de distillation (50), le procédé comportant avantageusement :
le passage d'au moins une partie de la fraction gazeuse aval (92) dans l'échangeur thermique aval (42) et/ou dans l'échangeur thermique intermédiaire (142, 146) et/ou dans l'échangeur thermique amont (34) pour y être réchauffée, le procédé comportant une étape de détente de la fraction gazeuse aval réchauffée dans une turbine de détente dynamique (152) ou dans une vanne de détente statique, avant d'être réintroduite dans l'échangeur thermique aval (42) et/ou dans l'échangeur thermique intermédiaire (142, 146) et/ou dans l'échangeur thermique amont (34).

9. Procédé selon la revendication 8, **caractérisé en ce qu'**il comprend une étape de circulation du flux aval (106) issu du courant de tête (98 ; 104) dans l'échangeur thermique amont (34) et/ou dans l'échangeur thermique intermédiaire (142, 146) et/ou dans l'échangeur thermique aval (42).

10. Procédé selon l'une quelconque des revendications précédentes, **caractérisé en ce qu'**au moins un flux de recirculation (112) issu du flux aval (106), après réchauffage du flux aval (106) dans l'échangeur thermique (34) est réintroduit après compression dans le courant de charge (12), en amont de l'étape de traitement (14).

11. Procédé selon l'une quelconque des revendications précédentes, **caractérisé en ce qu'**au moins un flux de recirculation (112) issu du flux aval (106), après réchauffage du flux aval (106) dans l'échangeur thermique (34) est réintroduit dans le courant de gaz traité (60), en aval de l'étape de traitement (14), pour former le courant intermédiaire (20).

## Patentansprüche

1. Verfahren zum Rückgewinnen eines Ethylenstroms (96) ausgehend von einem Ladestrom (12), welches die folgenden Schritte aufweist:
- Behandeln des Ladestroms (12) zum Erlangen eines behandelten Gasstroms (60, 70),
- Abkühlen und wenigstens partielles Kondensieren des behandelten Gasstroms (60, 70) in wenigstens einem Wärmetauscher (34, 42, 34, 142, 146, 42) zum Bilden von wenigstens einer Kolonneneinspeisungsfraktion,
- Einführen der oder jeder Kolonneneinspeisungsfraktion in eine Destillationskolonne (50), um einen Ethylenstrom (96) am Fuß der Destillationskolonne (50) und einen Kopf-Strom (98, 104), der arm an Ethylen ist, am Kopf rückzugewinnen,
- Erwärmen wenigstens einer Stromabwärts-Strömung (106), die vom Kopf-Strom (104) stammt, in einem Wärmetauscher (34, 42, 34, 142, 146, 42),
**dadurch gekennzeichnet, dass** der Schritt des Behandeins das Bilden eines Intermediär-Stroms (20) aufweist, der wenigstens 20 Mol-% Ethylen und wenigstens 20 Mol-% Kohlenstoffmonoxid aufweist, wobei das Verfahren einen Schritt des Eliminierens des Kohlenstoffmonoxids aufweist, das im Intermediär-Strom (20) enthalten ist, und dadurch, dass der Ethylenstrom (96) mehr als 99 Gew.-% Ethylen aufweist, das im Ladestrom (12) anwesend ist, wobei der Ethylenstrom (96) am Fuß der Destillationskolonne (50) ohne Passieren durch eine andere Destillationskolonne erlangt wird.

2. Verfahren gemäß Anspruch 1, **dadurch gekennzeichnet, dass** der Druck des behandelten Gasstroms, der in dem oder jedem Wärmetauscher (34, 42, 34, 142, 146, 42) zirkuliert, größer als 40 kgf/cm² ist und insbesondere zwischen 20 kgf/cm² und 40 kgf/cm² liegt,
wobei der Druck des behandelten Gasstroms in dem oder jedem Wärmetauscher (34, 42, 34, 142, 146, 42) vorteilhafterweise grundsätzlich kleiner als 30 kgf/cm² ist.

3. Verfahren gemäß irgendeinem der vorangegangenen Ansprüche, **dadurch gekennzeichnet, dass** der Ethylenstrom (96) einen Masseteil an Ethylen größer als 99,5% aufweist, wobei der Ethylenstrom (96) am Fuß der Destillationskolonne (50) ohne Passieren durch eine andere Destillationskolonne erlangt wird.

4. Verfahren gemäß irgendeinem der vorangegangenen Ansprüche, **dadurch gekennzeichnet, dass**, anschließend an den Schritt des Behandelns, der Intermediär-Strom (20) in eine Vorrichtung (24) zum Eliminieren von Kohlenstoffmonoxid ohne Destillation eingeführt wird, um einen Strom (62), der reich an Kohlenstoffmonoxid ist, und einen Strom (64), der arm an Kohlenstoffmonoxid ist, zu bilden, wobei der Druck des Stroms (64), der arm an Kohlenstoffmonoxid ist, vorteilhafterweise kleiner als 5 kgf/cm² ist, wobei die Vorrichtung (24) zum Eliminieren des Kohlenstoffmonoxids vorteilhafterweise ein Gerät zur Adsorption durch Druckmodulation aufweist, produzierend den Strom (62), der reich an Kohlenstoffmonoxid ist, und den Strom (64), der arm an Kohlenstoff ist.

5. Verfahren gemäß Anspruch 4, **dadurch gekennzeichnet, dass** der Strom (64), der arm an Kohlenstoffmonoxid ist, komprimiert wird, bis ein Rekompressionsdruck in einer Komprimierungsvorrichtung (26) zum Bilden des behandelten Gasstroms (70) vorteilhafterweise kleiner als 40 kgf/cm² ist.

6. Verfahren gemäß irgendeinem der Ansprüche 4 bis 5, **dadurch gekennzeichnet, dass** wenigstens eine Fraktion (72) des behandelten Gasstroms (60, 70) Wärmetausch mit der Stromabwärts-Strömung (106) in einem Stromaufwärts-Wärmetauscher (34) gesetzt wird, um einen partiell kondensierten Stromaufwärts-Strom (80) zu erlangen,
wobei der Stromaufwärts-Strom (80) in einen Stromaufwärts-Separator (40) eingeführt wird, wobei mindestens eine flüssige Fraktion (82), die vom Stromaufwärts-Separator (40) stammt, in die Destillationskolonne (50) gebracht wird.

7. Verfahren gemäß irgendeinem der vorangegangenen Ansprüche, **dadurch gekennzeichnet, dass** der Intermediär-Strom (20) direkt durch den behandelten Gasstrom (60) gebildet wird, dann in eine Einheit (16) der Kondensation und der Fraktionierung eingeführt wird, die eine Mehrzahl an Wärmetauschern (34, 142, 146, 42) aufweist, die bei abnehmenden Temperaturen funktionieren, um eine Mehrzahl an Einspeisungsfraktionen der Destillationskolonne (50) zu erzeugen, die in die Destillationskolonne (50) eingeführt werden, wobei der Kopf-Strom (104), der von der Destillationskolonne (50) stammt, mehr als 10 Gew.-% Kohlenstoffmonoxid aufweist, das in dem Intermediär-Strom (20) enthalten ist.

8. Verfahren gemäß Anspruch 7, **dadurch gekennzeichnet, dass** es die folgenden Schritte aufweist:
- Einführen des behandelten Gasstroms (60) in wenigstens einen ersten Stromaufwärts-Wärmetauscher (34), um einen Stromaufwärts-Strom (80) zu erlangen, der bei einer Temperatur von vorteilhafterweise kleiner als -50 °C abgekühlt und partiell kondensiert ist, und Trennen des Stromaufwärts-Stroms (80) in eine flüssige Stromaufwärts-Kolonneneinspeisungsfraktion (82) und in eine gasförmige Stromaufwärts-Fraktion (84),
- Zuführen der gasförmigen Stromaufwärts-Fraktion (84) in wenigstens einen ersten Intermediär-Wärmetauscher (142, 144, 146), um wenigstens einen Intermediär-Strom (162, 170) zu erlangen, der bei einer Temperatur von vorteilhafterweise kleiner als - 90 °C abgekühlt und partiell kondensiert ist, und Trennen des oder jedes abgekühlten Intermediär-Stroms (162, 170) in eine flüssige Intermediär-Kolonneneinspeisungsfraktion und eine gasförmige Intermediär-Fraktion (166, 174),
- Einführen von wenigstens einer gasförmigen Intermediär-Fraktion (174) in einen Stromabwärts-Wärmetauscher (42), um einen Stromabwärts-Strom (88), der bei einer Temperatur von vorteilhafterweise kleiner als -110 °C abgekühlt und partiell kondensiert ist, und Trennen des Stromabwärts-Stroms (88) in eine flüssige Stromabwärts-Kolonneneinspeisungsfraktion (90) und in eine gasförmige Stromabwärts-Fraktion (92), wobei die flüssigen Stromabwärts-, Stromaufwärts- oder Intermediär-Fraktionen in die Destillationskolonne (50) gebracht werden, das Verfahren vorteilhafterweise aufweisend:
- Passieren von wenigstens einem Teil der gasförmigen Stromabwärts-Fraktion (92) in den Stromabwärts-Wärmetauscher (42) und/oder in den Intermediär-Wärmetauscher (142, 146) und/oder in den Stromaufwärts-Wärmetauscher (34), um dort aufgewärmt zu werden, wobei das Verfahren einen Schritt des Entspannens der gasförmigen, aufgewärmten Stromabwärts-Fraktion in einer dynamischen Entspannungsturbine (152) oder in einem statischen Entspannungsventil aufweist, bevor sie in den Stromabwärts-Wärmetauscher (42) und/oder in den Intermediär-Wärmetauscher (142, 146) und/oder in den Stromaufwärts-Wärmetauscher (34) wiedereingeführt wird.

9. Verfahren gemäß Anspruch 8, **dadurch gekennzeichnet, dass** es einen Schritt des Zirkulierens der Stromabwärts-Strömung (106), die vom Kopf-Strom (98, 104) stammt, im Stromaufwärts-Wärmetauscher (34) und/oder im Intermediär-Wärmetauscher (142, 146) und/oder im Stromabwärts-Wärmetauscher (42) aufweist.

10. Verfahren gemäß irgendeinem der vorangegangenen Ansprüche, **dadurch gekennzeichnet, dass** eine Rezirkulierungsströmung (112), die von einer Stromabwärts-Strömung (106) stammt, nach dem Aufwärmen der Stromabwärts-Strömung (106) im Wärmetauscher (34) nach dem Komprimieren stromaufwärts des Schritts des Behandeins (14) wieder in den Ladestrom (12) eingeführt wird.

11. Verfahren gemäß irgendeinem der vorangegangenen Ansprüche, **dadurch gekennzeichnet, dass** wenigstens eine Rezirkulierungsströmung (112), die von der Stromabwärts-Strömung (106) stammt, nach dem Wiederaufwärmen der Stromabwärts-Strömung (106) in einem Wärmetauscher (34) wieder in den behandelten Gasstrom (60) stromaufwärts des Schritts des Behandeins (14) eingeführt wird, um den Intermediär-Strom (20) zu bilden.

## Claims

1. A method to recover an ethylene stream (96) from a feed stream (12), comprising the following steps:
- treating a feed stream (12) to obtain a treated gas stream (60; 70);
- at least partly cooling and condensing the treated gas stream (60 ; 70) in at least one heat exchanger (34, 42; 34, 142, 146, 42) to form at least one column feed fraction;
- feeding the or each column feed fraction into a distillation column (50) to recover an ethylene stream (96) at the foot of the distillation column and, at the head, a head stream (98; 104) depleted of ethylene;
- heating at least one downstream flow (106) derived from the head stream (104) in the heat exchanger (34, 42; 34, 142, 146, 42);
**characterized in that** the treatment step comprises the forming of an intermediate stream (20) containing at least 20 mole % ethylene and at least 20 mole % carbon monoxide, the method comprising a step to remove the carbon monoxide contained in the intermediate stream (20), and **in that** the ethylene stream (96) contains more than 99 weight % of the ethylene contained in the feed stream (12), the ethylene stream (96) being obtained at the foot of the distillation column (50) without passing through another distillation column.

2. The method according to claim 1, **characterized in that** the pressure of the treated gas stream circulating in the or in each heat exchanger (34, 42; 34, 142, 146, 42) is lower than 40 kgf/cm², in particular it is between 20 kgf/cm² and 40 kgf/cm²,
the pressure of the treated gas stream circulating in the or in each heat exchanger (34, 42; 34, 142, 146, 42) is strictly lower than 30 kgf/cm².

3. The method according to any of the preceding claims, **characterized in that** the ethylene stream (96) has an ethylene weight content higher than 99.5 %, the ethylene stream (96) being obtained at the foot of the distillation column (50) without passing through another distillation column.

4. The method according to any of the preceding claims, **characterized in that**, at the treatment step, the intermediate stream (20) is led into a device (24) to remove carbon monoxide without distillation to form a carbon monoxide-rich stream (62) and a carbon monoxide-depleted stream (64), the pressure of the carbon monoxide-depleted stream (64) advantageously being lower than 5 kgf/cm², the carbon monoxide removing device (24) comprising a pressure swing adsorber (28) producing the carbon monoxide-rich stream (62) and the carbon monoxide-depleted stream (64).

5. The method according to claim 4, **characterized in that** the carbon monoxide-depleted stream (64) is compressed to a recompression pressure advantageously lower than 40 kgf/cm², in compression equipment (26) to form the treated gas stream (70).

6. The method according to any of claims 4 to 5, **characterized in that** at least one fraction (72) of the treated gas stream (60; 70) is placed under heat exchange with the downstream flow (106) in an upstream heat exchanger (34), to obtain a partly condensed upstream stream (80),
the upstream stream (80) being led into an upstream separator (40) and at least one liquid fraction (82) output from the upstream separator (40) being led into the distillation column (50).

7. The method according to any of the preceding claims, **characterized in that** the intermediate stream (20) is formed directly by the treated gas stream (60) and then led into a condensation and fractionating assembly (16) comprising a plurality of heat exchangers (34, 142, 146, 42) operating at decreasing temperatures to produce a plurality of feed fractions for the distillation column (50) fed into the distillation column (50), the head stream (104) output from the distillation column (50) comprising more than 10 weight % of the carbon monoxide contained in the intermediate stream (20).

8. The method according to claim 7, **characterized in that** it comprises the following steps:
- leading the treated gas stream (60) into at least one first upstream heat exchanger (34) to obtain a cooled and partly condensed upstream stream (80) at a temperature advantageously lower than -50 °C, and separating the upstream stream (80) into an upstream column feed liquid fraction (82) and an upstream gas fraction (84);
- leading the upstream gas fraction (84) into at least one intermediate heat exchanger (142, 144, 146) to obtain at least one cooled and partly condensed intermediate stream (162, 170) at a temperature advantageously lower than -90 °C, and separating the or each cooled intermediate stream (162, 170) into an intermediate column feed liquid fraction and an intermediate gas fraction (166, 174);
- leading at least one intermediate gas fraction (174) into a downstream heat exchanger (42) to obtain a cooled and partly condensed downstream stream (88) at a temperature advantageously lower than -110 °C, and separating the downstream stream (88) into a downstream column feed liquid fraction (90) and a downstream gas fraction (92), the upstream, downstream and intermediate liquid fractions being fed into the distillation column (50), the method advantageously comprising:
passing at least part of the downstream gas fraction (92) through the downstream heat exchanger (42) and/or through the intermediate heat exchanger (142, 146) and/or through the upstream heat exchanger (34) to be heated therein, the method comprising a step to expand the heated downstream gas fraction in a dynamic expansion turbine (152) or static pressure-reducing valve before being sent back to the downstream heat exchanger (42) and/or intermediate heat exchanger (142, 146) and/or upstream heat exchanger (34).

9. The method according to claim 8, **characterized in that** it comprises a step to circulate the downstream flow (106) derived from the head stream (98; 104) in the upstream heat exchanger (34) and/or in the intermediate heat exchanger (142, 146) and/or in the downstream heat exchanger (42).

10. The method according to any of the preceding claims, **characterized in that** at least one recirculation flow (112) derived from the downstream flow (106), after heating the downstream flow (106) in the heat exchanger (34) is fed back after compression into the feed stream (12), upstream of the treatment step (14).

11. The method according to any of the preceding claims, **characterized in that** at least one recirculation flow (112) derived from the downstream flow (106), after heating the downstream flow (106) in the heat exchanger (34), is fed back into the treated gas stream (60), downstream of the treatment step (14), to form the intermediate stream (20).
